# EUROPÄISCHE PATENTSCHRIFT

(11) **EP 1 419 432 B1**
(45) Veröffentlichungstag und Bekanntmachung des Hinweises auf die Patenterteilung: **04.05.2011**
(21) Anmeldenummer: 02796210.9
(22) Anmeldetag: 09.08.2002
(51) Int. Cl.: G06F 3/048

(54) **VORRICHTUNG ZUR BEDIENUNG EINES MEDIZINISCHEN GERÄTES, INSBESONDERE EINES BEWEGLICHEN OPERATIONSTISCHES**
DEVICE FOR OPERATING A MEDICAL APPLIANCE, ESPECIALLY A MOBILE OPERATING TABLE
DISPOSITIF POUR LA COMMANDE D'UN APPAREIL MEDICAL, NOTAMMENT D'UNE TABLE D'OPERATION MOBILE

(30) Priorität: 24.08.2001 DE 10142738
(43) Veröffentlichungstag der Anmeldung: 19.05.2004
(73) Patentinhaber: Storz Endoskop Produktions GmbH, 78532 Tuttlingen (DE)
(72) Erfinder: NOVAK, Pavel, CH-8207 Schaffhausen (CH); STROEHLE, Tasso, 88605 Messkirch (DE)
(74) Vertreter: Duhme, Torsten
(86) Internationale Anmeldenummer: PCT/EP2002/008900
(87) Internationale Veröffentlichungsnummer: WO 2003/018087

(56) Entgegenhaltungen:
- WO-A-94/22069
- DE-A- 19 929 907
- DE-A- 19 955 116
- DE-U- 8 804 417
- GB-A- 2 269 252
- US-A- 4 651 365
- US-A- 4 914 624
- US-A- 5 890 178
- US-A- 6 131 868

## Beschreibung

Die vorliegende Erfindung betrifft eine Vorrichtung zur Bedienung eines medizinischen Gerätes, insbesondere eines beweglichen Operationstisches, mit einem Touch-Screen, auf dem eine Bedienoberfläche dargestellt ist, und mit einer Steuereinheit, wobei die Bedienoberfläche eine Anzahl von ersten Schaltflächen aufweist, und wobei die Steuereinheit Aktionen des medizinischen Gerätes bewirkt, wenn die ersten Schaltflächen betätigt werden, wobei die Bedienoberfläche ferner zumindest einen visuell hervorgehobenen Stoppbereich aufweist, bei dessen Betätigung jede Aktion gestoppt wird.

Eine Vorrichtung dieser Art ist aus DE 197 14 984 C2 bekannt.

Die Verwendung von Touch-Screens zur Bedienung und Steuerung von Geräten ist vor allem im medizinischen Bereich mittlerweile weit verbreitet. Bei einem Touch-Screen handelt es sich um einen Bildschirm oder allgemeiner um eine Anzeigeeinheit mit einer berührungsempfindlichen Oberfläche. Ein Touch-Screen ermöglicht daher die bildliche Darstellung von (virtuellen) Schaltflächen, deren Berührung durch einen Bediener erkannt und ausgewertet werden kann. Mit einem Touch-Screen lassen sich daher sehr flexible und vor allem auch sehr leicht und intuitiv zu bedienende Eingabegeräte realisieren.

Für manche Geräte, gerade im medizinischen Bereich, ist jedoch eine besonders zuverlässige und sichere Bedienung erforderlich. So muß bspw. die Bewegung eines elektrischen Operationstisches sehr zuverlässig gesteuert werden können, um einen schwerverletzten Patienten vorsichtig in eine gewünschte Lage zu bringen. Dabei muß sichergestellt sein, daß die Bewegung des Operationstisches jederzeit zuverlässig wieder gestoppt werden kann. Diese auf den ersten Blick selbstverständliche Eigenschaft ist mit den bekannten Touch-Screens nicht ohne weiteres sichergestellt. Es ist nämlich nicht auszuschließen, daß die virtuelle Schaltfläche des Touch-Screens nach ihrer Betätigung "hängenbleibt", d. h. daß aufgrund einer Fehlfunktion des Touch-Screens das Loslassen der berührungsempfindlichen Schaltfläche nicht mehr registriert wird. Wenn mit der Schaltfläche in einem solchen Fall die Bewegung des Operationstisches gestartet wurde, kann die Bewegung über das Touch-Screen nicht mehr zuverlässig gestoppt werden. Aus diesem Grund wurde bislang davon abgesehen, kritische Aktionen bei medizinischen Geräten, d. h. Aktionen, die mit einer sehr hohen Zuverlässigkeit durchgeführt werden müssen, allein mit Hilfe von Touch-Screens zu steuern. Zum Teil wurde die Bedienung derartiger Aktionen ganz ohne Touch-Screen realisiert, oder das Touch-Screen wurde wie bei der eingangs genannten Vorrichtung um einen zusätzlichen "echten" Notaus-Schalter ergänzt.

Das Hinzufügen eines Notaus-Schalters erfordert jedoch eine zusätzliche Verdrahtung sowie einen Eingriff in die Hardware von handelsüblichen Touch-Screens. Dies ist im Hinblick auf die Herstellungskosten einer gattungsgemäßen Vorrichtung, aber auch im Hinblick auf den Bedienungskomfort von Nachteil.

Eine alternative Möglichkeit, um mit einem Touch-Screen eine zuverlässige und fehlersichere Steuerung zu realisieren, besteht darin, die einzelnen Steuerungsvorgänge bei Betätigung der Schaltfläche jeweils nur für eine festgelegte, begrenzte Zeitspanne durchzuführen. Hierdurch ist gewährleistet, daß bspw. die Bewegung des Operationstisches nach Ablauf der festgelegten Zeitspanne gestoppt wird, selbst wenn die Schaltfläche des Touch-Screens aufgrund eines zwischenzeitlich aufgetretenen Fehlers hängenbleibt. Eine solche Maßnahme ist aus DE 199 29 907 A1 bekannt. Sie schränkt jedoch den Bedienungskomfort ein. Darüber hinaus ist es dann sehr schwierig, eine definierte Position des Operationstisches mit hinreichender Präzision einzustellen.

Aus GB 2 269 252 A ist ein Treppenlift bekannt, bei dem am oberen und unteren Ende der Treppe eine Bedieneinheit mit Bedientasten angeordnet ist. Die Bedieneinheit besitzt zwei nebeneinanderliegende Tasten zum Hoch- oder Runterfahren des Treppenliftes. Unterhalb dieser beiden Tasten ist eine Not-Stopp-Taste angeordnet.

Aus DE 88 04 417 U1 ist eine Bedieneinheit für ein Bett bekannt. Diese Bedieneinheit besitzt vier Tasten, mit denen man das Kopfteil und das Fußteil auf- bzw. abbewegen kann. Unterhalb der vier Tasten ist ebenfalls eine Not-Stopp-Taste angeordnet. Ebenso wie bei der Bedieneinheit der GB 2 269 252 A sind die Bedien- und Not-Stopp-Tasten allerdings mechanische Taster. Ein Touch-Screen ist hier nicht offenbart.

Aus US 4, 914, 624 ist es bekannt, dass man auf einem resistiven Touch-Screen virtuelle Tastendrücke erzeugen kann, indem man den Touch-Screen an zwei verschiedenen Stellen betätigt. Der resistive Touch-Screen liefert in diesem Fall eine Betätigungsposition, die in etwa mittig zwischen den tatsächlichen Berührungspunkten liegt. US 4,914,624 schlägt vor, diese Eigenschaft zu verwenden, um eine erste Betätigung des Touch-Screens an einer ersten Position in gewisser Weise zu neutralisieren.

Vor diesem Hintergrund ist es eine Aufgabe der vorliegenden Erfindung, eine Vorrichtung der eingangs genannten Art anzugeben, die eine komfortable und zugleich sichere und zuverlässige Bedienung des medizinischen Gerätes ermöglicht.

Diese Aufgabe wird mit einer Vorrichtung nach Anspruch 1 gelöst.

Die erfindungsgemäße Vorrichtung macht sich den Effekt zunutze, daß die Auswerteelektronik handelsüblicher Touch-Screens dann, wenn mehrere Punkte auf dem Touch-Screen gleichzeitig betätigt werden, deren Mittelpunkt als virtuellen Berührungspunkt auswertet. Dies trifft zumindest für die sog. resistiven Touch-Screens zu, die für medizinische Anwendungen üblicherweise eingesetzt werden. Der ausgewertete Mittelpunkt bei gleichzeitiger Berührung des Touch-Screens an mehreren Stellen wird z. T. auch als geometrischer Mittelpunkt bezeichnet.

Die erfindungsgemäße Verteilung der ersten Schaltflächen und des Stoppbereichs hat zur Folge, daß die Betätigung einer ersten Schaltfläche selbst dann aufgehoben werden kann, wenn der Touch-Screen aufgrund eines Fehlers an der betätigten ersten Schaltfläche "hängenbleibt". Berührt man nämlich anschließend den Touch-Screen im Stoppbereich, erkennt die Auswerteelektronik als virtuellen Berührungspunkt diejenige Stelle, die mittig zwischen der hängengebliebenen ersten Schaltfläche und dem späteren Berührungspunkt im Stoppbereich liegt. Damit kann durch eine zweite Berührung des Touch-Screens der von der Auswerteteelektronik erkannte Berührungspunkt aus dem Bereich der ersten Schaltfläche "herausgezogen" werden. Für die Auswerteelektronik bzw. die nachgeschaltete Steuereinheit entspricht dies der Situation, daß die erste Schaltfläche wieder losgelassen wurde. Somit erkennt die Steuereinheit das Loslassen der ersten Schaltfläche spätestens dann, wenn der Bediener den Touch-Screen in dem genannten Stoppbereich berührt.

Ein "Hängenbleiben" des Touch-Screens im Stoppbereich ist nicht kritisch, da dies automatisch zur Folge hat, daß sämtliche kritischen Aktionen gestoppt werden. Infolgedessen ist durch die erfindungsgemäße Verteilung der ersten Schaltflächen und des Stoppbereichs sichergestellt, daß eine Aktion, die durch Betätigen einer ersten Schaltfläche ausgelöst wird, jederzeit wieder gestoppt werden kann, und zwar zumindest durch die erneute Betätigung des Touch-Screens im Stoppbereich. Der Stoppbereich erfüllt somit die Funktion eines Notaus-Schalters, so daß ein zusätzlicher hardwaremäßiger Notaus-Schalter entfallen kann.

Die erfindungsgemäße Verteilung der ersten Schaltflächen und des Stoppbereichs hat darüber hinaus den Vorteil, daß eine Betätigung des Touch-Screens im Stoppbereich stets zu einem definierten Abschalten der kritischen Aktion führt. Da alle denkbaren Mittelpunkte zwischen dem Stoppbereich und den ersten Schaltflächen außerhalb jeder ersten Schaltfläche liegen, wird bei Betätigung des Touch-Screens im Stoppbereich auch keine unerwünschte neue Aktion ausgelöst. Die Gefahr eines "Ping-pong"-Effektes, bspw. zwischen verschiedenen Bewegungsrichtungen des Operationstisches, ist dadurch vermieden. Außerdem ist auf diese Weise auch gewährleistet, daß eine kritische Bewegung nicht durch eine abrupte Gegenbewegung gestoppt wird, was für einen Patienten u. U. noch schlimmere Folgen haben könnte.

Durch die erfindungsgemäße Anordnung der ersten Schaltflächen und des Stoppbereichs wird eine sehr kostengünstige und gleichzeitig sehr komfortable Möglichkeit bereitgestellt, auch kritische Aktionen bei medizinischen Geräten zuverlässig und sicher zu steuern. Darüber hinaus ist aufgrund der Tatsache, daß sämtliche Bedienvorgänge einschließlich der Notstopp-Funktion über den Touch-Screen ausgelöst werden, eine einheitliche und durchgängige Bedienung gewährleistet. Dies ist insbesondere in Notfall-Situationen von Vorteil, da auf diese Weise nicht erst an ungewohnten Stellen nach dem richtigen Schalter gesucht werden muß.

In einer Ausgestaltung der Erfindung beinhaltet der Stoppbereich eine zweite Schaltfläche.

Durch diese Maßnahme wird der Stoppbereich für den Bediener des medizinischen Gerätes visuell erkennbar gemacht. Für die eigentliche Stopp-Funktion ist dies von der technischen Seite her gesehen nicht zwingend notwendig. Da die Auswerteelektronik des Touch-Screens in jedem Fall den Mittelpunkt zwischen mehreren gleichzeitigen Berührungspunkten als virtuellen Berührungspunkt erkennt, kann eine hängengebliebene erste Schaltfläche grundsätzlich durch jede beliebige Berührung an einer anderen Stelle aufgehoben werden. Die visuelle Hervorhebung des Stoppbereichs besitzt jedoch den Vorteil, daß die Notstopp-Funktion das medizinische Gerät in einen definierten Ruhezustand bringt, der für den Bediener vorhersehbar ist. Unbeabsichtigte, abrupte Gegenbewegungen werden dadurch zuverlässiger vermieden.

In einer weiteren Ausgestaltung bewirkt die Steuereinheit der Vorrichtung eine erste Aktion nur während der Betätigung der zugehörigen ersten Schaltfläche.

Diese Maßnahme hat zur Folge, daß eine ausgelöste Aktion im Normalbetrieb der Vorrichtung sofort beendet wird, wenn der Bediener die zugehörige erste Schaltfläche losläßt. Die Notstopp-Funktion ist daher tatsächlich nur für Notfälle erforderlich, in denen die erste Schaltfläche hängenbleibt. Die besondere Funktion des Stoppbereichs wird dadurch stärker hervorgehoben, was die intuitive Bedienung des medizinischen Gerätes erleichtert. Darüber hinaus besitzt die Maßnahme den Vorteil, daß die Notstopp-Funktion technisch noch einfacher realisiert werden kann, da die Betätigung des Touch-Screens im Stoppbereich, wie zuvor beschrieben, zur Folge hat, daß sich der vom Touch-Screen erkannte Berührungspunkt aus dem Bereich der ersten Schaltfläche hinaus verschiebt. Infolgedessen führt die genannte Maßnahme in Kombination mit der erfindungsgemäßen Verteilung der Schaltflächen automatisch zur Beendigung der ausgelösten Aktion.

In einer weiteren Ausgestaltung sind die ersten Aktionen räumliche Bewegungen des medizinischen Gerätes.

Die erfindungsgemäße Anordnung der ersten Schaltflächen und des Stoppbereichs ist besonders vorteilhaft, wenn man räumliche Bewegungen von medizinischen Geräten, wie bspw. von einem Operationstisch oder von einem Instrumentenroboter, steuern möchte. Derartige Aktionen wurden bislang aufgrund der bestehenden Bedenken nicht mit Touch-Screens gesteuert, es sei denn, daß die Touch-Screens über zusätzliche hardwaremäßige Abschaltwege verfügten. Die Vorteile der Erfindung kommen daher besonders beim Steuern räumlicher Bewegungen von medizinischen Geräten zur Geltung.

In einer weiteren Ausgestaltung der Erfindung beinhaltet die Steuereinheit eine Spracheingabeeinheit zum Aufnehmen und Auswerten von Sprachbefehlen.

Durch diese Maßnahme wird der Bediendungskomfort der Vorrichtung weiter erhöht. Dem Bediener des medizinischen Gerätes stehen so verschiedene intuitive Bedienmöglichkeiten zur Verfügung.

In einer weiteren Ausgestaltung der zuvor genannten Maßnahme sind die Spracheingabeeinheit und die Bedienoberfläche derart miteinander gekoppelt, daß zumindest der Stoppbereich bei jeder Eingabe eines Sprachbefehls aktiv ist.

Diese Maßnahme besitzt den Vorteil, daß auch die Sprachsteuerung des medizinischen Gerätes nicht durch zusätzliche hardwaremäßige Notaus-Schalter abgesichert werden muß. Die Funktion des Notaus-Schalters kann nämlich aufgrund der zuvor beschriebenen Zusammenhänge in zuverlässiger und gleichzeitig kostengünstiger Weise durch den Stoppbereich auf dem Touch-Screen realisiert werden.

Besonders vorteilhaft ist es, wenn die Bedienoberfläche mit den ersten Schaltflächen und dem Stoppbereich automatisch aktiviert wird, sobald ein Sprachbefehl eingegeben wird, dessen Funktion der Betätigung einer ersten Schaltfläche entspricht.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

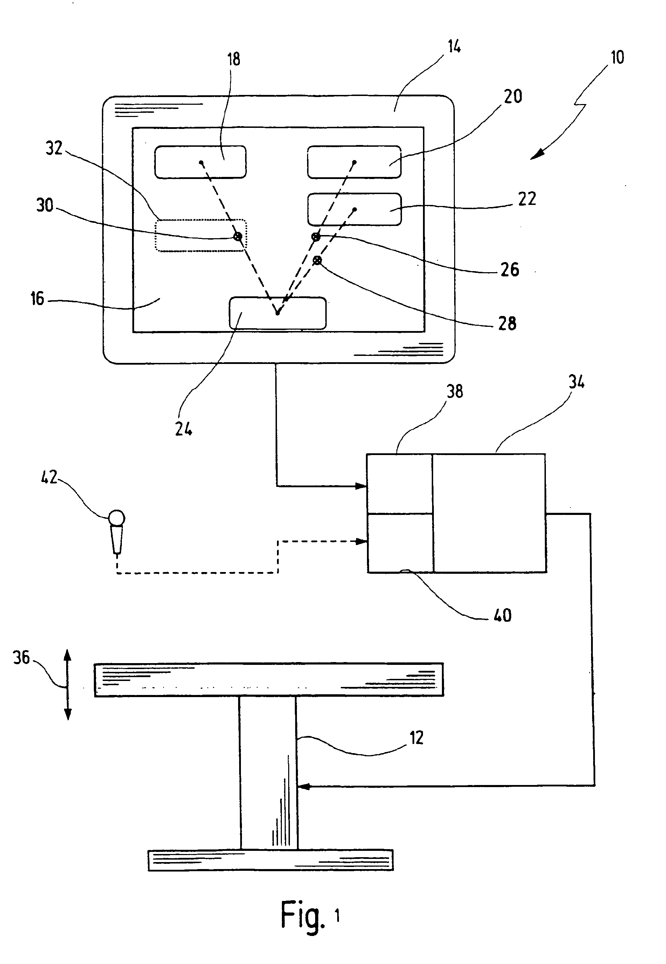

Ausführungsbeispiele der Erfindung sind in der Zeichnung dargestellt und werden in der nachfolgenden Beschreibung näher erläutert.

In der einzigen Figur ist eine schematische Darstellung eines Ausführungsbeispiels der Erfindung gezeigt. Dabei ist die erfindungsgemäße Vorrichtung in ihrer Gesamtheit mit der Bezugsziffer 10 bezeichnet.

Die Vorrichtung 10 dient in dem dargestellten Ausführungsbeispiel zur Bedienung eines elektrisch beweglichen Operationstisches 12. Die Erfindung ist jedoch nicht auf diesen Anwendungsfall beschränkt und kann ebenso bspw. zur Bedienung eines Roboterarms oder zur Bedienung von Insufflatoren, Pumpen und dgl. verwendet werden.

Die Vorrichtung 10 beinhaltet einen an sich bekannten resistiven Touch-Screen 14, auf dem eine Bedienoberfläche 16 dargestellt ist. Die Bedienoberfläche 16 weist in dem hier gezeigten schematischen Ausführungsbeipiel drei erste Schaltflächen 18, 20, 22 auf. Darüber hinaus ist auf der Bedienoberfläche 16 hier eine zweite Schaltfläche 24 dargestellt. Die Schaltfläche 24 liegt vollständig in einem Stoppbereich, der sich durch seine nachfolgend näher beschriebene räumliche Beziehung zu den ersten Schaltflächen 18 bis 22 bestimmt.

Mit den Bezugsziffern 26, 28, 30 sind die geometrischen Mittelpunkte zwischen jeder ersten Schaltfläche 18, 20, 22 und der zweiten Schaltfläche 24 bezeichnet. Bildlich gesehen liegen die Mittelpunkte 26, 28, 30 jeweils mittig auf einer Verbindungslinie zwischen den Flächenschwerpunkten der ersten Schaltflächen 18, 20, 22 und der zweiten Schaltfläche 24.

Wie in der Darstellung zu erkennen ist, liegen die Mittelpunkte 26, 28, 30 außerhalb jeder ersten Schaltfläche 18, 20, 22. Zur Verdeutlichung dieser Eigenschaft ist mit der Bezugsziffer 32 die Position einer (nicht vorhandenen) ersten Schaltfläche bezeichnet, die der erfindungsgemäßen Verteilung widerspräche. Die Anordnung einer Schaltfläche an der Position 32 hätte nämlich zur Folge, daß durch die Betätigung der zweiten Schaltfläche 24 (Notaus-Schalter) eine unerwünschte Aktion ausgelöst würde, wenn die Schaltfläche 18 aufgrund eines Fehlers des Touch-Screens 14 "hängenbleiben" würde. Da die Mittelpunkte 26, 28 demgegenüber in einem völlig neutralen Bereich der Bedienoberfläche 16 liegen, hätte eine Betätigung der zweiten Schaltfläche 24 in Kombination mit einer hängengebliebenen ersten Schaltfläche 20 oder 22 keine unerwünschte Aktion zur Folge. Es würde lediglich die mit der hängengebliebenen Schaltfläche 20, 22 verbundene Aktion gestoppt.

Mit der Bezugsziffer 34 ist schematisch eine Steuereinheit bezeichnet, die den Operationstisch 12 in Richtung des Pfeils 36 nach oben bzw. nach unten bewegen kann. Derartige Steuereinheiten sind bei gattungsgemäßen Vorrichtungen an sich bekannt, so daß sich eine nähere Beschreibung hier erübrigt.

Mit der Bezugsziffer 38 ist schematisch die Auswerteelektronik des Touch-Screens 14 bezeichnet. Der Einfachheit halber ist die Auswerteelektronik 38 hier dem Bereich der Steuereinheit 34 zugeordnet. In der Praxis ist die Auswerteelektronik 38 demgegenüber häufig in das Gehäuse des Touch-Screens 14 integriert. Die Auswerteelektronik 38 wertet in an sich bekannter Weise Berührungen im Bereich der Bedienoberfläche 16 des Touch-Screens 14 aus und liefert der Steuereinheit 34 Datenwerte, die den erkannten Berührungspositionen entsprechen. Die Steuereinheit verarbeitet die erhaltenen Datenwerte und steuert in Abhängigkeit davon den Operationstisch 12 an.

Mit der Bezugsziffer 40 ist eine Spracheingabeeinheit bezeichnet, die wiederum der Einfachheit halber der Steuereinheit 34 zugeordnet ist. Alternativ kann die Spracheingabeeinheit 14 auch getrennt von der Steuereinheit 34 angeordnet sein und bspw. über Kabel mit dieser verbunden sein. Die Spracheingabeeinheit 40 erhält ihre Sprachsignale in an sich bekannter Weise über ein Mikrofon 42. Die Sprachsteuerung des Operationstisches 12 ist in dem hier dargestellten Ausführungsbeispiel eine optionale, zusätzliche Variante. Aus diesem Grund ist die Anbindung des Mikrofons 42 an die Steuereinheit 34 nur gestrichelt dargestellt.

Gemäß einem bevorzugten Ausführungsbeispiel der Erfindung ist die Steuereinheit 34 ein an sich handelsüblicher Computer, der mit einer speziellen Steuerungssoftware zum Durchführen der benötigten Steuerungsfunktionen programmiert ist. Die Software beinhaltet dabei einen vorrichtungsinternen Funktionstest, der zunächst nach der Inbetriebnahme der Vorrichtung ausgeführt werden muß. Im Rahmen dieses Funktionstests wird u. a. die Funktionsfähigkeit des Touch-Screens 14 überprüft. Werden zu diesem Zeitpunkt Fehler festgestellt, wird der Betrieb der Vorrichtung 10 eingeschränkt oder ganz unterbunden. Ein vollständiger Betrieb der Vorrichtung 10 ist nur möglich, wenn der Eingangstest fehlerfrei durchlaufen wird.

Die Software der Steuereinheit 34 beinhaltet darüber hinaus, daß nach dem Auslösen einer kritischen Aktion, bspw. der Bewegung 36 des Operationstisches 12, jede Berührung des Touch-Screens 14 außerhalb der ersten Schaltfläche, die der ausgelösten Funktion entspricht, zu einem Stoppbefehl führt. Die Betätigung des Touch-Screens 14 im Bereich der zweiten Schaltfläche 24 führt daher automatisch zu einem Stopp der Bewegung des Operationstisches 12, und zwar selbst dann, wenn die zugehörige erste Schaltfläche nach der Betätigung hängenbleibt. Dies gilt aufgrund der genannten Ausführung selbst dann, wenn die Bewegung des Operationstisches 12 über die Sprachsteuerung ausgelöst wurde. Die zweite Schaltfläche 24 hat damit die Funktion eines generellen Notstopp-Schalters. Im Normalbetrieb der Vorrichtung 10 wird die Bewegung des Operationstisches 12 demgegenüber bereits beendet, wenn die zugehörige betätigte erste Schaltfläche losgelassen wird.

## Patentansprüche

1. Vorrichtung zur Bedienung eines medizinischen Gerätes, insbesondere eines beweglichen Operationstisches (12), mit einem Touch-Screen (14), auf dem eine Bedienoberfläche (16) dargestellt ist, und mit einer Steuereinheit (34), wobei die Bedienoberfläche (16) eine Anzahl von ersten Schaltflächen (18, 20, 22) aufweist, und wobei die Steuereinheit (34) Aktionen (36) des medizinischen Gerätes (12) bewirkt, wenn die ersten Schaltflächen (18, 20, 22) betätigt werden, **dadurch gekennzeichnet, dass** die Bedienoberfläche (16) ferner zumindest einen visuell hervorgehobenen Stoppbereich (24) aufweist, bei dessen Betätigung jede Aktion (36) gestoppt wird, dass der Touch-Screen (14) eine Auswerteelektronik, aufweist, die dafür ausgebildet ist, wenn mehrere Punkte auf dem Touch-Screen (14) gleichzeitig betätigt werden, deren Mittelpunkt als virtuellen Berührungspunkt auszuwerten, dass die ersten Schaltflächen (18, 20, 22) und der Stoppbereich (24) räumlich derart auf der Bedienoberfläche (16) verteilt sind, dass alle denkbaren Mittelpunkte (26, 28, 30) einer Verbindungslinie zwischen Flächenschwerpunkten jeder ersten Schaltfläche (18, 20, 22) und dem Stoppbereich (24) außerhalb jeder ersten Schaltfläche (18, 20, 22) liegen und so eine Betätigung des Touch-Screens (14) im Stoppbereich (24) stets zu einem definierten Abschalten der Aktionen (36) führt.

2. Vorrichtung nach Anspruch 1, **dadurch gekennzeichnet, dass** der Stoppbereich eine zweite Schaltfläche (24) beinhaltet.

3. Vorrichtung nach Anspruch 1 oder 2, **dadurch gekennzeichnet, dass** die Steuereinheit (34) eine Aktion (36) nur während der Betätigung der zugehörigen ersten Schaltfläche (18, 20, 22) bewirkt.

4. Vorrichtung nach einem der Ansprüche 1 bis 3, **dadurch gekennzeichnet, dass** die Aktionen (36) räumliche Bewegungen des medizinischen Gerätes (12) sind.

5. Vorrichtung nach einem der Ansprüche 1 bis 4, **dadurch gekennzeichnet, dass** die Steuereinheit (34) eine Spracheingabeeinheit (40) zum Aufnehmen und Auswerten von Sprachbefehlen beinhaltet.

6. Vorrichtung nach Anspruch 5, **dadurch gekennzeichnet, dass** die Spracheingabeeinheit (40) und die Bedienoberfläche (16) derart miteinander gekoppelt sind, dass zumindest der Stoppbereich (24) bei jeder Eingabe eines Sprachbefehls aktiv ist.

## Claims

1. Apparatus for operating a medical device, in particular a movable operating table (12), comprising a touch-screen (14), on which a user interface (16) is being displayed, and comprising a control unit (34), wherein the user interface (16) comprises a number of first buttons (18, 20, 22), and wherein the control unit (34) triggers actions (36) of the medical device (12) if the first buttons (18, 20, 22) are pressed, **characterized in that** the user interface (16) further comprises at least one visually emphasized stop-area (24), which, when actuated, stops any action (36), that the touch-screen (14) comprises an evaluation electronics adapted to evaluate, if multiple points on the touch-screen (14) are touched simultaneously, their center as a virtual point of touch, and that the first buttons (18, 20, 22) and the stop-area (24) are distributed on the user interface (16) such that all possible centers (26, 28, 30) of a connecting line between the centers of area of each first button (18, 20, 22) and the stop-area (24) lie outside of each button (18, 20, 22) so that an actuation of the touch-screen (14) in the stop-area (24) always leads to a defined stop of the actions (36).

2. Apparatus according to claim 1, **characterized in that** the stop-area comprises a second button (24).

3. Apparatus according to claim 1 or 2, **characterized in that** the control unit (34) performs an action (36) only during the actuation of the corresponding first button (18, 20, 22).

4. Apparatus according to any of claims 1 to 3, **characterized in that** the actions (36) are movements in space of the medical device (12).

5. Apparatus according to any of claims 1 to 4, **characterized in that** the control unit (34) comprises a speech input unit (40) for recording and evaluating speech commands.

6. Apparatus according to claim 5, **characterized in that** the speech input unit (40) and the user interface (16) are linked such that at least the stop-button (24) is active during each input of a speech command.

## Revendications

1. Dispositif destiné à faire fonctionner un appareil médical, notamment une table d'opération mobile (12), comprenant un écran tactile (14) sur lequel est affichée une interface utilisateur (16), et comprenant une unité de commande (34), dans lequel l'interface utilisateur (16) présente une pluralité de premiers boutons (18, 20, 22), et dans lequel l'unité de commande (34) effectue des actions (36) de l'appareil médical (12) lorsque les premiers boutons (18, 20, 22) sont actionnés, **caractérisé en ce que** l'interface utilisateur (16) comprend en outre au moins une zone d'arrêt ressortant visuellement (24) qui interrompt chaque action (36) lorsqu'elle est actionnée, **en ce que** l'écran tactile (14) comprend des moyens électroniques d'évaluation qui sont conçus de telle manière que lorsqu'on appuie simultanément sur plusieurs points de l'écran tactile (14) dont le centroïde est évalué comme étant un point de contact virtuel, les premiers boutons (18, 20, 22) et la zone d'arrêt (24) soient répartis dans l'espace sur l'interface utilisateur (16) de telle façon que tous les centroïdes possibles (26, 28, 30) d'une ligne de jonction entre des centroïdes de chacun des premiers boutons (18, 20, 22) et de la zone d'arrêt (24) se situent à l'extérieur de chaque premier bouton (18, 20, 22) et ainsi, qu'un actionnement de l'écran tactile (14) dans la zone d'arrêt (24) conduise au lieu de cela à une interruption des actions (36).

2. Dispositif selon la revendication 1, **caractérisé en ce que** la zone d'arrêt comporte un deuxième bouton (24).

3. Dispositif selon la revendication 1 ou 2, **caractérisé en ce que** l'unité de commande (34) n'effectue une action (36) que pendant l'actionnement du premier bouton (18, 20, 22) correspondant.

4. Dispositif selon l'une des revendications 1 à 3, **caractérisé en ce que** les actions (36) sont des mouvements dans l'espace de l'appareil médical (12).

5. Dispositif selon l'une des revendications 1 à 4, **caractérisé en ce que** l'unité de commande (34) comporte une unité d'entrée vocale (40) destinée à recevoir et interpréter des ordres vocaux.

6. Dispositif selon la revendication 5, **caractérisé en ce que** l'unité d'entrée vocale (40) et l'interface utilisateur (16) sont couplées l'une à l'autre de telle manière que la zone d'arrêt (24) soit active lors de chaque entrée d'un ordre vocal.
